# EUROPEAN PATENT SPECIFICATION

(11) **EP 4 361 124 B1**
(45) Date of publication and mention of the grant of the patent: **09.12.2026**
(21) Application number: 23837534.9
(22) Date of filing: 18.05.2023
(51) Int. Cl.: C07C 51/377, C07C 51/43, C07C 51/44, C07C 57/04, C07C 59/08

(54) **METHOD FOR PREPARING ACRYLIC ACID**
VERFAHREN ZUR HERSTELLUNG VON ACRYLSÄURE
PROCÉDÉ DE PRÉPARATION D'ACIDE ACRYLIQUE

(30) Priority: 14.09.2022 KR 20220115905
(43) Date of publication of application: 01.05.2024
(73) Proprietor: LG CHEM, LTD., Seoul 07336 (KR)
(72) Inventor: LYU, Byeong Gil, Daejeon 34122 (KR); KIM, Mi Kyung, Daejeon 34122 (KR); SHIN, Dae Young, Daejeon 34122 (KR); JUNG, Da Bin, Daejeon 34122 (KR)
(74) Representative: Goddar, Heinz J.
(86) International application number: PCT/KR2023/006774
(87) International publication number: WO 2024/058338

(56) References cited:
- WO-A1-2022/103087
- JP-A- 2009 242 285
- JP-A- 2014 189 510
- KR-A- 20160 122 749
- KR-A- 20170 113 177
- KR-A- 20220 078 233

## Description

### [Technical Field]

The present invention relates to a method for preparing acrylic acid, and more particularly, to a method for effectively removing by-products while reducing loss of acrylic acid in preparing acrylic acid through a dehydration reaction of lactic acid.

### [Background Art]

Acrylic acid is used as a polymer raw material used in fibers, adhesives, paints, fiber processing, leather, construction materials, and the like, and its demand is expanding. In addition, the acrylic acid is also used as a raw material of an absorbent resin, and is widely used industrially for absorbent products such as paper diapers and sanitary napkins, water-retaining agents for agricultural and horticultural use, and waterstops for industrial use.

As a method for preparing acrylic acid according to the related art, a method of air oxidizing propylene is generally used, but this method is a method for preparing acrylic acid by converting propylene into acrolein by a gas-phase catalytic oxidation reaction, and subjecting the acrolein to a gas-phase catalytic oxidation reaction, and in this case, acetic acid is produced as a by-product, which is difficult to separate from acrylic acid. In addition, the method for preparing acrylic acid using propylene uses, as a raw material, propylene obtained by refining crude oil, which is a fossil resource, and has problems in terms of raw material costs or environmental pollution considering problems such as the recent rise in crude oil prices or global warming.

Accordingly, studies on a method for preparing acrylic acid from a carbon-neutral biomass raw material have been conducted. For example, there is a method for preparing acrylic acid (AA) through a gas-phase dehydration reaction of lactic acid (LA). This method is a method for preparing acrylic acid through an intramolecular dehydration reaction of lactic acid generally at a high temperature of 300°C or higher in the presence of a catalyst. A reaction product including acrylic acid is produced through the dehydration reaction of lactic acid, and unreacted lactic acid is included in the reaction product according to a conversion rate. In a case where unreacted lactic acid is included in the reaction product, the economic efficiency of the process may be improved only when the unreacted lactic acid is recovered in a separation process. However, an oligomerization reaction of the lactic acid proceeds rapidly at a high concentration and a high temperature, and it is difficult to recover the lactic acid.

WO 2022/103087 A1 discloses a method for preparing acrylic acid by dehydrating lactic acid molecules.

JP 2014189510 A discloses a method for producing acrylic acid including a reaction step of forming acrylic acid by dehydration reaction of lactic acid to obtain a reaction product gas, a condensation step of condensing the reaction product gas and one or more distillation steps.

### [Disclosure]

### [Technical Problem]

In order to solve the problems mentioned in the background art, an object of the present invention is to provide a method for effectively separating unreacted lactic acid from a reaction product produced in preparing acrylic acid through a dehydration reaction of lactic acid, and saving energy consumption.

### [Technical Solution]

In one general aspect, a method for preparing acrylic acid includes: obtaining a reaction product including unreacted lactic acid, water, a light gas component, and acrylic acid by supplying a lactic acid aqueous solution to a reactor to allow a dehydration reaction to proceed; supplying the reaction product to a first cooling tower to separate the reaction product into a lower fraction containing unreacted lactic acid and an upper fraction containing water, a light gas component, and acrylic acid; supplying the upper fraction of the first cooling tower to a second cooling tower to separate a lower fraction containing water and acrylic acid therefrom; obtaining acrylic acid by purifying the lower fraction of the second cooling tower, wherein an operating temperature of the first cooling tower is 100°C to 180°C, wherein an operating temperature of the second cooling tower is 60°C to 140°C, and wherein the dehydration reaction is performed as a gas phase reaction in the presence of a catalyst.

Further embodiments are disclosed in the dependent claims.

### [Advantageous Effects]

According to the method for preparing acrylic acid of the present invention, unreacted lactic acid is separated in advance before distillation of the reaction product including acrylic acid, such that energy costs may be reduced compared to the case of separating unreacted lactic acid after distillation.

In particular, prior to distillation of the reaction product, an acrylic acid aqueous solution having a composition favorable to a subsequent acrylic acid purification is formed using two cooling towers, such that energy costs required for purifying acrylic acid may be reduced, and at the same time, high-purity acrylic acid may be obtained.

In addition, the acrylic acid aqueous solution discharged from the cooling tower is divided and supplied to an extraction column and an azeotropic distillation column, such that energy consumption required for distillation of water in the azeotropic distillation column may be saved, and at the same time, loss of acrylic acid may be reduced and high-purity acrylic acid may be obtained.

### [Description of Drawings]

FIG. 1 is a process flow chart according to a method for preparing acrylic acid in an exemplary embodiment of the present invention.
FIG. 2 is a process flow chart according to a method for preparing acrylic acid according to a comparative example.

### [Detailed Description]

The terms and words used in the description and claims of the present invention are not to be construed limitedly as having general or dictionary meanings but are to be construed as having meanings and concepts meeting the technical ideas of the present invention, based on a principle that the inventors are able to appropriately define the concepts of terms in order to describe their own inventions in the best mode.

The term "stream" in the present invention may refer to a flow of a fluid in a process, or may refer to a fluid itself flowing in a pipe. Specifically, the stream may refer to both a fluid itself flowing in a pipe connecting respective devices to each other and a flow of a fluid. In addition, the fluid may refer to a gas or liquid, and a case of the fluid including a solid component is not excluded.

Meanwhile, in the present invention, in devices such as a cooling tower, an extraction column, and a distillation tower, the "lower portion" of the device refers to a point at a height of 95% to 100% from the top of the device to the bottom, unless otherwise specified, and specifically, may refer to the bottom (of the tower). Similarly, the "upper portion" of the device refers to a point at a height of 0% to 5% from the top of the device to the bottom, unless otherwise specified, and specifically, may refer to the top (of the tower).

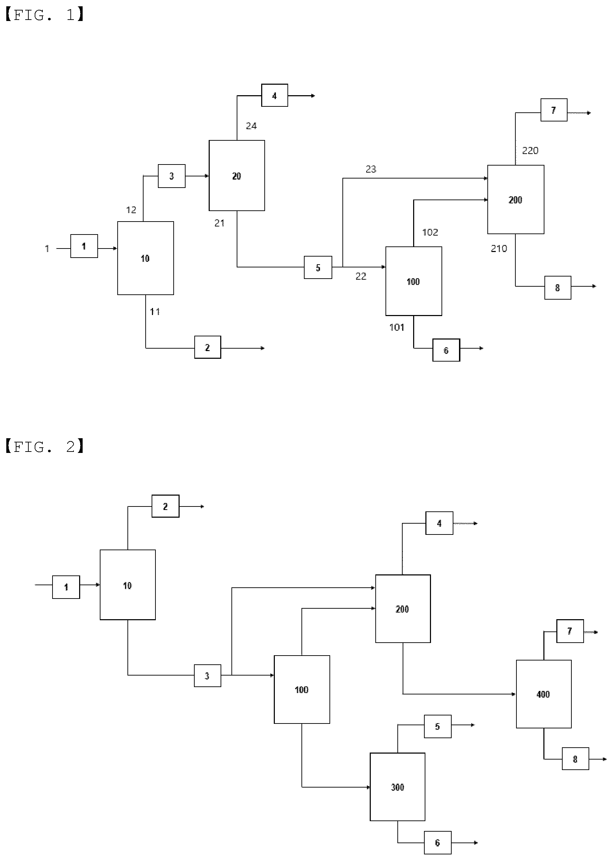

In addition, unless otherwise specified, an operating temperature of the cooling tower in the present invention may refer to an operating temperature at the lower portion of the cooling tower, and an operating pressure of the cooling tower may refer to an operating pressure at the upper portion of the cooling tower. Hereinafter, each process that may be included in exemplary embodiments of the present invention will be described with reference to FIG. 1 and the like.

A method for preparing acrylic acid according to the present invention includes: obtaining a reaction product including unreacted lactic acid, water, a light gas component, and acrylic acid by supplying a lactic acid aqueous solution to a reactor to allow a dehydration reaction to proceed; supplying the reaction product to a first cooling tower to separate the reaction product into a lower fraction containing unreacted lactic acid and an upper fraction containing water, a light gas component, and acrylic acid; supplying the upper fraction of the first cooling tower to a second cooling tower to separate a lower fraction containing water and acrylic acid therefrom; and obtaining acrylic acid by purifying the lower fraction of the second cooling tower, wherein an operating temperature of the first cooling tower is 100°C to 180°C, wherein an operating temperature of the second cooling tower is 60°C to 140°C, and wherein the dehydration reaction is performed as a gas phase reaction in the presence of a catalyst.

First, the method for preparing acrylic acid according to the present invention includes obtaining a reaction product including unreacted lactic acid, water, a light gas component, and acrylic acid by supplying a lactic acid aqueous solution to a reactor to allow a dehydration reaction to proceed.

Specifically, as a method for preparing acrylic acid according to the related art, a method of air oxidizing propylene is generally used, but this method is a method for preparing acrylic acid by converting propylene into acrolein by a gas-phase catalytic oxidation reaction, and subjecting the acrolein to a gas-phase catalytic oxidation reaction, and in this case, acetic acid is produced as a by-product, which is difficult to separate from acrylic acid. In addition, the method for preparing acrylic acid using propylene uses, as a raw material, propylene obtained by refining crude oil, which is a fossil resource, and has problems in terms of raw material costs or environmental pollution considering problems such as the recent rise in crude oil prices or global warming.

In order to solve the problems of the method for preparing acrylic acid according to the related art, studies on a method for preparing acrylic acid from a carbon-neutral biomass raw material have been conducted. For example, there is a method for preparing acrylic acid (AA) through a gas-phase dehydration reaction of lactic acid (LA). This method is a method for preparing acrylic acid through an intramolecular dehydration reaction of lactic acid generally at a high temperature in the presence of a catalyst. A reaction product including acrylic acid is produced through the dehydration reaction of lactic acid, and unreacted lactic acid is included in the reaction product according to a conversion rate. In a case where unreacted lactic acid is included in the reaction product, the economic efficiency of the process may be improved only when the unreacted lactic acid is recovered in a separation process. However, an oligomerization reaction of the lactic acid proceeds rapidly at a high concentration and a high temperature, and it is difficult to recover the lactic acid. Furthermore, in a case where an additional distillation tower or the like to recover lactic acid is provided, the overall process cost increases because a lot of energy is required for operation.

Accordingly, in order to solve the problems in the related art, the present invention is intended to provide a method capable of improving a recovery rate of unreacted lactic acid and reducing costs for equipment such as an additional distillation device for separating lactic acid in a subsequent process and operating costs for its operation by separating lactic acid from a reaction product including acrylic acid produced through a dehydration reaction of lactic acid in advance before a distillation process so that the time when lactic acid at a high concentration is exposed to a high temperature is shortened to prevent oligomerization of lactic acid.

According to an exemplary embodiment of the present invention, a reaction product including acrylic acid may be produced by supplying a lactic acid aqueous solution to a reactor in advance and allowing a dehydration reaction to proceed. In this case, the dehydration reaction is performed as a gas phase reaction in the presence of a catalyst. For example, a concentration of lactic acid in the lactic acid aqueous solution may be 10 wt% or more, 20 wt% or more, or 30 wt% or more, and 40 wt% or less, 50 wt% or less, 60 wt% or less, or 70 wt% or less. When the lactic acid is present at a high concentration, oligomers such as dimers and trimers may be formed by an equilibrium reaction, and therefore, lactic acid may be used in the form of an aqueous solution at a concentration within the above range.

The reactor may include a reactor capable of dehydrating normal lactic acid, the reactor may include a reaction tube filled with a catalyst, and acrylic acid may be produced by dehydrating lactic acid by a gas-phase catalytic reaction while passing a reaction gas including volatile components of a lactic acid aqueous solution as a raw material through the reaction tube. In addition to lactic acid, the reaction gas may further include one or more diluent gases selected from water vapor, nitrogen, and air for concentration adjustment.

The operation of the reactor may be performed under normal lactic acid dehydration reaction conditions. In this case, an operating temperature of the reactor may refer to a set temperature of a heat medium or the like used for temperature control of the reactor.

The catalyst used in the dehydration reaction of lactic acid may include, for example, one or more selected from the group consisting of a sulfate-based catalyst, a phosphate-based catalyst, and a nitrate-based catalyst. As specific examples, the sulfate may include Na₂SO₄, K₂SO₄, CaSO₄, and Al₂(SO₄)₃, the phosphate may include Na₃PO₄, Na₂HPO₄, NaH₂PO₄, K₃PO₄, K₂HPO₄, KH₂PO₄, CaHPO₄, Ca₃(PO₄)₂, AlPO₄, CaH₂P₂O₇, and Ca₂P₂O₇, and the nitrate may include NaNO₃, KNO₃, and Ca(NO₃)₂. In addition, the catalyst may be supported on a support. Examples of the support include one or more selected from the group consisting of diatomite, alumina, silica, titanium dioxide, carbide, and zeolite.

The reaction product produced through the dehydration reaction of lactic acid may include water (H₂O), a light gas component, and unreacted lactic acid in addition to acrylic acid, which is a desired product.

The method for preparing acrylic acid through a dehydration reaction of lactic acid may secure raw material competitiveness compared to the method of air oxidizing propylene according to the related art and may solve the problem of environmental pollution, but a conversion rate of lactic acid is low, and various by-products are produced, resulting in a low yield of acrylic acid. Therefore, development of a process for improving economic efficiency is demanded. Accordingly, the present invention may provide a method for improving economic efficiency by reducing overall equipment costs and energy costs as well as increasing a recovery rate of unreacted lactic acid.

The method for preparing acrylic acid according to the present invention includes supplying the reaction product to a first cooling tower to separate the reaction product into a lower fraction containing unreacted lactic acid and an upper fraction containing water, a light gas component, and acrylic acid.

Specifically, a reactor discharge stream 1 containing the reaction product is a gaseous stream, and the reactor discharge stream 1 may be supplied to the first cooling tower 10 to be cooled. That is, unreacted lactic acid having a relatively high boiling point in the gaseous reaction product supplied to the first cooling tower 10 is cooled and condensed to form a liquid condensate, and may be separated into the lower fraction of the first cooling tower 10. Meanwhile, components other than unreacted lactic acid in the reaction product, specifically, water, a light gas component, and acrylic acid may be separated into the upper fraction of the first cooling tower 10 as gaseous phases. Here, the light gas component is a component having a lower boiling point than water, and specifically, may include carbon monoxide, carbon dioxide, and acetaldehyde in addition to the diluent gas.

Unreacted lactic acid included in the reaction product is separated in advance by cooling, such that deformation of lactic acid due to exposure to a high temperature, for example, oligomerization, may be prevented to increase a recovery rate of lactic acid, and the recovered lactic acid may be efficiently reused as a raw material for the dehydration reaction for preparing acrylic acid. In addition, since there is no need to provide an additional distillation device or the like for separating and recovering unreacted lactic acid in a subsequent process, energy costs for operating the distillation device may be reduced. Furthermore, unreacted lactic acid is separated before an azeotropic distillation process described below, such that energy consumption required for azeotropic distillation may be saved.

To this end, an operating temperature of the first cooling tower 10 is 100°C to 180 °C 110 °C or higher, or 120 °C or higher, 170°C or lower, or 160°C or lower. When the temperature is lower than 100°C, components other than unreacted lactic acid may be excessively condensed, and the purity of the recovered lactic acid is reduced, which may cause loss of acrylic acid, which is a desired product. On the other hand, when the temperature exceeds 180°C, unreacted lactic acid is not sufficiently condensed, which may cause discharge of unreacted lactic acid through the upper of the first cooling tower 10, and as a result, a recovery rate of lactic acid may be reduced, and it may be difficult to obtain high-purity acrylic acid.

In addition, an operating pressure of the first cooling tower 10 may be 98 kPa (1 kg/cm²) or more, 147 kPa (1.5 kg/cm²) or more, or 176.4 kPa (1.8 kg/cm²) or more, and 1960 kPa (20 kg/cm²) or less, 980 kPa (10 kg/cm²) or less, or 490 kPa (5 kg/cm²) or less. When the pressure is high, a volumetric flow rate is reduced, resulting in a reduction in cost of the cooling tower, but dimers of lactic acid and acrylic acid may be produced due to an increase in operating temperature of the cooling tower, and therefore, it is required to set an appropriate operating pressure at which a dimer is not produced.

When the operating conditions of the first cooling tower 10 are controlled to the operating temperature and the operating pressure within the above ranges, compositions of a lower discharge stream and an upper discharge stream of the first cooling tower 10 may be controlled, and through this, a composition of an acrylic acid aqueous solution stream discharged through the lower of the second cooling tower 20 may be easily controlled.

From this point of view, the upper fraction discharged from the first cooling tower may not contain unreacted lactic acid, and even when unreacted lactic acid is contained, unreacted lactic acid may be contained in an amount of 5 wt% or less, and specifically, 3 wt% or less.

Meanwhile, a ratio of a flow rate of water in the stream discharged through the lower of the first cooling tower to a flow rate (kg/hr) of water included in the reaction product introduced into the first cooling tower 10 may be 15 wt% or less, and a ratio of a flow rate of acrylic acid in the stream discharged through the lower of the first cooling tower to a flow rate (kg/hr) of acrylic acid included in the reaction product introduced into the first cooling tower 10 may be 15 wt% or less.

In the method for preparing acrylic acid according to the related art, even when a cooling tower is used, the cooling tower is used for cooling the gaseous reaction product so that the gaseous reaction product is appropriately introduced into an acrylic acid purification process, and is not a cooling tower used for material separation. This is because it is difficult to recover a material with a desired purity when the material is separated by cooling. However, in the present invention, the cooling amount of the cooling tower is controlled to adjust the amount of components to be cooled, such that the material separation effect may also be obtained, in addition to cooling of the reaction product, which is the original object of the present invention.

Meanwhile, the upper fraction containing water, a light gas component, and acrylic acid may be supplied to the second cooling tower 20 as an upper discharge stream 12 of the first cooling tower 10. Meanwhile, the lower fraction containing unreacted lactic acid may be discharged as a lower discharge stream 11 of the first cooling tower 10, and lactic acid recovered from the lower discharge stream 11 may be supplied to the reactor and may be reused in a dehydration reaction of lactic acid.

Subsequently, the upper discharge stream 12 of the first cooling tower 10 supplied to the second cooling tower 20 is additionally cooled, such that the upper discharge stream 12 may be separated into a lower fraction containing water and acrylic acid and an upper fraction containing a light gas component.

An operating temperature of the second cooling tower 20 is 60°C to 140 °C, 80°C or higher, or 100°C or higher, and 130°C or lower, or 120°C or lower, and an operating pressure of the second cooling tower 20 may be 78.4 kPa (0.8 kg/cm²) or more, 98 kPa (1.0 kg/cm²) or more, or 127.4 kPa (1.3 kg/cm²) or more, and 1960 kPa (20 kg/cm²) or less, 980 kPa (10 kg/cm²) or less, or 490 kPa (5 kg/cm²) or less. When the operating conditions of the second cooling tower 20 are controlled to the operating temperature and the operating pressure within the above ranges, a composition of a light gas component separated through an upper discharge stream 24 of the second cooling tower 20 is controlled, such that the loss of acrylic acid may be minimized, a light gas component containing a diluent gas and acetaldehyde may be removed out of the system, and a composition of an acrylic acid aqueous solution stream 21 containing acrylic acid and water discharged through the lower of the second cooling tower 20 may be controlled.

Meanwhile, the acrylic acid aqueous solution stream 21 derived from the lower fraction of the second cooling tower 20 containing water and acrylic acid may be introduced into a purification process for obtaining acrylic acid. The purification process is a process for obtaining high-purity acrylic acid from water and some impurities in the acrylic acid aqueous solution, and the obtained acrylic acid should be recovered with high purity, and energy consumption involved in the process should be saved in consideration of economic efficiency.

For example, the purification process may be performed by an extraction process of separating the acrylic acid aqueous solution into an extract containing acrylic acid and an extraction solvent and a raffinate containing water using an extraction solvent in an extraction column. However, when the purification process is performed by an extraction process, although energy consumption may be more reduced than in the distillation process, it may be difficult to obtain high-purity acrylic acid because some by-products to be removed as well as water are contained in the extract.

Meanwhile, as another example of the purification process, the purification process may be performed by an azeotropic distillation process. In this case, on the premise of using an azeotropic solvent, the separation efficiency between water and acrylic acid is higher than in a simple extraction process, and thus, high-purity acrylic acid may be obtained, but excessive energy consumption is required due to accompanying distillation of water having a high specific heat, which may be less preferable in terms of economic efficiency.

Therefore, according to an exemplary embodiment of the present invention, the purification process may be performed in combination with the extraction process and the azeotropic distillation process by supplying a part of the acrylic acid aqueous solution stream 21 to an extraction column 100 as a first acrylic acid aqueous solution stream 22, and supplying a remainder to an azeotropic distillation column 200 as a second acrylic acid aqueous solution stream 23. That is, the acrylic acid aqueous solution stream 21 is divided and supplied to the extraction column 100 and the azeotropic distillation column 200, such that energy consumption in a subsequent process may be saved, and by-products that may be partially contained in the acrylic acid aqueous solution stream 21 may be efficiently separated.

Specifically, a ratio of a flow rate of the first acrylic acid aqueous solution stream 22 supplied to the extraction column to the total flow rate of the first acrylic acid aqueous solution stream 21 before branching, that is, the first acrylic acid aqueous solution stream and the second acrylic acid aqueous solution stream after branching, may be 30 wt% to 70 wt%, and specifically, 40 wt% to 50 wt%. When the flow rate ratio is 30 wt% or more, the flow rate introduced into the azeotropic distillation column 200 is reduced, and the amount of energy consumed in distilling water having a high specific heat in the azeotropic distillation column 200 may be reduced. Meanwhile, when the flow rate ratio is 70 wt% or less, by-products may be efficiently separated through the upper of the azeotropic distillation column 200, such that accumulation of the by-products in the system may be prevented, high-purity acrylic acid may also be obtained, the amount of extractant required for removing water in the extraction column 100 may be reduced, a flow rate of the extractant introduced into the azeotropic distillation column 200 may be reduced to reduce the amount of energy consumed in distillation, and the by-products may be efficiently separated, thereby obtaining high-purity acrylic acid.

Meanwhile, the extraction column 100 removes most of the water contained in the first acrylic acid aqueous solution stream 22 without using a lot of energy and supplies the first acrylic acid aqueous solution stream 22 from which water is mostly removed to the azeotropic distillation column 200, such that energy used for azeotropic distillation in the azeotropic distillation column 200 described below may be reduced. In this respect, it is preferable that, in the extraction in the extraction column 100, the extraction solvent comes into contact with the stream supplied from the extraction column by a liquid-liquid contact method from the viewpoint of improving the energy efficiency of the entire process.

In this case, the extraction solvent may be a hydrocarbon-based solvent that may form an azeotrope with water and does not form an azeotrope with acrylic acid, but may sufficiently extract acrylic acid, and it is advantageous in the extraction process that the extraction solvent has a boiling point of 10 to 120°C. Specifically, the extraction solvent may be one or more solvents selected from the group consisting of benzene, toluene, xylene, n-heptane, cycloheptane, cycloheptene, 1-heptene, ethyl-benzene, methyl-cyclohexane, n-butyl acetate, isobutyl acetate, isobutyl acrylate, n-propyl acetate, isopropyl acetate, methyl isobutyl ketone, 2-methyl-1-heptene, 6-methyl-1-heptene, 4-methyl-1-heptene, 2-ethyl-1-hexene, ethylcyclopentane, 2-methyl-1-hexene, 2,3-dimethylpentane, 5-methyl-1-hexene, and isopropyl-butyl-ether.

In addition, as the extraction column 100, an extraction device based on a liquid-liquid contact method may be used. Non-limiting examples of the extraction device include a Karr reciprocating plate column, a rotary-disk contactor, a Scheibel column, a Kuhni column, a spray extraction tower, a packed extraction tower, a pulsed packed column, a mixer-settler bank, a mixer, and a centrifuge (centrifugal counter current extractor).

With such a method, water in the first acrylic acid aqueous solution stream 22 supplied to the extraction column 100 may be significantly removed, an extract containing an extraction solvent and acrylic acid may be obtained, and the extract may be supplied to the azeotropic distillation column 200 as an upper discharge stream 102 of the extraction column.

In addition, water contained in the first acrylic acid aqueous solution stream 22 may be recovered as a raffinate by the extraction process. The recovered raffinate may be discharged as a lower discharge stream 101 of the extraction column. As water is recovered in the extraction process as described above, the energy consumption may be significantly saved by reducing an operating load of a distillation process described below.

Subsequently, according to an exemplary embodiment of the present invention, the second acrylic acid aqueous solution stream 23 and the upper discharge stream 102 of the extraction column may be supplied to the azeotropic distillation column 200, and a distillation process for these streams may be performed. The distillation process in the azeotropic distillation column 200 for the stream supplied to the azeotropic distillation column 200 may be a process of separating the stream into an upper fraction containing water and an extraction solvent and a lower fraction containing acrylic acid by azeotropic distillation.

According to the present invention, it is advantageous in terms of process that the distillation in the azeotropic distillation column 200 is performed in the presence of an azeotropic solvent. Here, the azeotropic solvent is a hydrophobic solvent that may form an azeotrope with water and does not form an azeotrope with acrylic acid, and any hydrocarbon-based solvent that satisfies the physical properties may be used without limitation. In addition, the azeotropic solvent may have a boiling point lower than that of acrylic acid, and preferably, may have a boiling point of 10 to 120°C.

According to the present invention, the azeotropic solvent that satisfies the physical properties may be one or more solvents selected from the group consisting of benzene, toluene, xylene, n-heptane, cycloheptane, cycloheptene, 1-heptene, ethyl-benzene, methyl-cyclohexane, n-butyl acetate, isobutyl acetate, isobutyl acrylate, n-propyl acetate, isopropyl acetate, methyl isobutyl ketone, 2-methyl-1-heptene, 6-methyl-1-heptene, 4-methyl-1-heptene, 2-ethyl-1-hexene, ethylcyclopentane, 2-methyl-1-hexene, 2,3-dimethylpentane, 5-methyl-1-hexene, and isopropyl-butyl-ether.

In addition, the azeotropic solvent may be the same as or different from the extract solvent applied to the extraction column 100. However, considering production efficiency and the like according to a continuous process, the azeotropic solvent is preferably the same as the extraction solvent. As described above, when the same compound is used as the azeotropic solvent and the extraction solvent, at least a part of the azeotropic solvent distilled and recovered in the azeotropic distillation column 200 may be supplied to the extraction column 100 and used as a part of the extraction solvent.

When the azeotropic solvent is added to the azeotropic distillation column 200 as described above, the azeotrope of acrylic acid and water is broken. Accordingly, water in the second acrylic acid aqueous solution stream 23 and the azeotropic solvent used in the azeotropic distillation may form an azeotrope together and may be recovered through an upper fraction of the azeotropic distillation column 200. In addition, a lower fraction 201 containing acrylic acid may be recovered through the lower of the azeotropic distillation column 200.

The upper fraction thus recovered of the azeotropic distillation column may be supplied to a layer separator through an upper discharge stream 202 of the azeotropic distillation column. The layer separator is a liquid-liquid layer separator and is a device for separating immiscible fluids using gravity or centrifugal force based on a density difference. A relatively light liquid may be separated through the upper of the layer separator, and a relatively heavy liquid may be separated through the lower of the layer separator. Specifically, the upper discharge stream 202 of the azeotropic distillation column supplied to the layer separator may be separated into an organic layer including an azeotropic solvent and a water layer including water.

In addition, the organic layer separated in the layer separator is discharged through a discharge stream of the layer separator, and the discharge stream of the layer separator containing an azeotropic solvent or an extraction solvent may be circulated to one or more of the extraction column and the azeotropic distillation column to be reused as an azeotropic solvent or an extraction solvent.

Hereinabove, the method for preparing acrylic acid according to the present invention has been described and illustrated in the drawings. However, the description and the illustration of the drawings are for only essential components for understating the present invention, and processes and devices not separately described and illustrated may be properly applicable and used for implementing the method for preparing acrylic acid according to the present invention, in addition to the processes and devices described and illustrated in the drawings.

Hereinafter, the present invention will be described in more detail by Examples.

### Examples

### Example 1

According to the process flow chart illustrated in FIG. 1, the acrylic acid producing process was simulated using Aspen Plus simulator available from Aspen Technology, Inc.

Specifically, 30 wt% of a lactic acid aqueous solution and nitrogen (N₂) as a diluent gas were supplied to a reactor, and a reaction product including unreacted lactic acid, water, a light gas component, and acrylic acid was produced through a dehydration reaction.

A reactor discharge stream containing the reaction product was supplied to a first cooling tower 10. In the first cooling tower 10, the reactor discharge stream was condensed and separated into a lower discharge stream 11 of the first cooling tower containing unreacted lactic acid and an upper discharge stream 12 of the first cooling tower containing water, a light gas component, and acrylic acid. At this time, a lower operating temperature and an upper operating pressure of the first cooling tower 10 were controlled to 125°C and 186.2 kPa (1.9 kg/cm²), respectively.

Subsequently, the upper discharge stream 12 of the first cooling tower was supplied to a second cooling tower 20 and then cooled and condensed, such that the upper discharge stream 12 of the first cooling tower was separated into a lower discharge stream 21 of the second cooling tower containing water and acrylic acid and an upper discharge stream 24 of the second cooling tower containing a light gas component including nitrogen. At this time, a lower operating temperature and an upper operating pressure of the second cooling tower 20 were controlled to 107°C and 127.4 kPa (1.3 kg/cm²), respectively.

A part of the lower discharge stream 21 of the second cooling tower was supplied to an extraction column 100 as a first acrylic acid aqueous solution stream 22, a remainder was supplied to an azeotropic distillation column 200 as a second acrylic acid aqueous solution stream 23, and a ratio of a mass flow rate of the first acrylic acid aqueous solution stream 22 supplied to the extraction column 100 to a flow rate of the lower discharge stream 21 of the second cooling tower was maintained at 50 wt%.

Meanwhile, in the extraction column 100, acrylic acid was dissolved using toluene as an extraction solvent, and then an extract containing acrylic acid and an extraction solvent was separated through an upper discharge stream 102 of the extraction column 100 and supplied to the azeotropic distillation column 200, and water was discharged through a lower discharge stream 101 of the extraction column 100.

In addition, distillation was performed in the azeotropic distillation column 200 to which the second acrylic acid aqueous solution stream 23 and the upper discharge stream 102 of the extraction column 100 were supplied, such that acrylic acid was obtained through the lower discharge, and a stream containing water and an extraction solvent was discharged through the upper. Subsequently, the stream containing water and an extraction solvent was supplied to a layer separator to separate the stream into water and an extraction solvent, water was discharged out of the system, and the extraction solvent was separately circulated to the extraction column 100 and the azeotropic distillation column 200.

In this case, the flow rate (kg/hr) and composition (wt%) of each component in each stream are shown in Table 1.

**[Table 1]**

| Component | Unit | 1 | 2 | 3 | 4 | 5 | 6 | 7 | 8 |
|---|---|---|---|---|---|---|---|---|---|
| Flow rate | | 11000 | 1362 | 9638 | 1023 | 8615 | 2949 | 3928 | 1738 |
| N₂ | kg/hr | 1000 | 0 | 1000 | 1000 | 0 | 0 | 0 | 0 |
| Lactic acid | kg/hr | 600 | 535 | 65 | 0 | 65 | 26 | 0 | 38 |
| Water | kg/hr | 7480 | 612 | 6868 | 22 | 6846 | 2917 | 3928 | 0 |
| Acrylic acid | kg/hr | 1920 | 214 | 1706 | 1 | 1705 | 5 | 0 | 1699 |
| Composition | | | | | | | | | |
| N₂ | wt% | 9% | 0% | 12% | 98% | 0% | 0% | 0% | 0% |
| Lactic acid | wt% | 5% | 39% | 1% | 0% | 1% | 1% | 0% | 2% |
| Water | wt% | 68% | 45% | 71% | 2% | 79% | 99% | 100% | 0% |
| Acrylic acid | wt% | 17% | 16% | 18% | 0% | 20% | 0% | 0% | 98% |

### Example 2

According to the process flow chart illustrated in FIG. 1, the acrylic acid producing process was simulated using Aspen Plus simulator available from Aspen Technology, Inc.

Specifically, a process was performed in the same manner as that of Example 1, except that 40 wt% of a lactic acid aqueous solution was supplied to a reactor, and the lower temperature of the first cooling tower 10 was controlled to 133°C.

In this case, the flow rate (kg/hr) and composition (wt%) of each component in each stream are shown in Table 2.

**[Table 2]**

| Component | Unit | 1 | 2 | 3 | 4 | 5 | 6 | 7 | 8 |
|---|---|---|---|---|---|---|---|---|---|
| Flow rate | | 11000 | 2729 | 8271 | 1023 | 7248 | 1742 | 3942 | 1567 |
| N₂ | kg/hr | 1000 | 0 | 1000 | 1000 | 0 | 0 | 0 | 0 |
| Lactic acid | kg/hr | 2000 | 1740 | 260 | 0 | 260 | 72 | 0 | 188 |
| Water | kg/hr | 6400 | 769 | 5631 | 22 | 5609 | 1669 | 3940 | 0 |
| Acrylic acid | kg/hr | 1600 | 220 | 1380 | 1 | 1379 | 0 | 0 | 1379 |
| Composition | | | | | | | | | |
| N₂ | wt% | 9% | 0% | 12% | 98% | 0% | 0% | 0% | 0% |
| Lactic acid | wt% | 18% | 64% | 3% | 0% | 4% | 4% | 0% | 12% |
| Water | wt% | 58% | 28% | 68% | 2% | 77% | 96% | 100% | 0% |
| Acrylic | wt% | 15% | 8% | 17% | 0% | 19% | 0% | 0% | 88% |
| acid | | | | | | | | | |

### Comparative Examples

### Comparative Example 1

According to the process flow chart illustrated in FIG. 2, the acrylic acid producing process was simulated using Aspen Plus simulator available from Aspen Technology, Inc.

In Comparative Example 1, a light gas component was separated using one cooling tower, and an additional distillation tower was used downstream of the extraction column and the azeotropic distillation column in order to implement the content of acrylic acid in the stream from which acrylic acid was recovered to 98 wt% as in Example 1.

As in Example 1, a reaction product was produced using a lactic acid aqueous solution. Specifically, 30 wt% of a lactic acid aqueous solution and nitrogen (N₂) as a diluent gas were supplied to a reactor, and a reaction product including unreacted lactic acid, water, a light gas component, and acrylic acid was produced through a dehydration reaction.

The reaction product was supplied to the cooling tower 10, a light gas component such as nitrogen was separated through the upper discharge, and a stream containing lactic acid, acrylic acid, and water was separated through the lower discharge. At this time, a lower operating temperature and an upper operating pressure of the cooling tower 10 were controlled to 108°C and 127.4 kPa (1.3 kg/cm²), respectively.

Thereafter, a part of a lower discharge stream of the cooling tower 10 was supplied to the extraction column 100, a remainder was supplied to the azeotropic distillation column 200, and a ratio of a mass flow rate of the acrylic acid aqueous solution stream supplied to the extraction column 100 to a flow rate of the lower discharge stream of the cooling tower was maintained at 50 wt%.

Meanwhile, in the extraction column 100, acrylic acid was dissolved using toluene as an extraction solvent, and then an extract containing acrylic acid and an extraction solvent was separated through the upper discharge stream of the extraction column 100 and supplied to the azeotropic distillation column 200. Subsequently, distillation was performed in the azeotropic distillation column 200, and the upper discharge stream of the extraction column 100 was separated into a lower discharge stream containing lactic acid and acrylic acid and an upper discharge stream containing water and an extraction solvent.

Lactic acid was contained in both the lower discharge stream of the extraction column 100 and the lower discharge stream of the azeotropic distillation column 200, and first and second lactic acid separation columns 300 and 400 had to be introduced in order to separate lactic acid contained in each stream.

Specifically, lactic acid and water contained in the lower discharge stream of the extraction column 100 were separated by the first lactic acid separation column 300 through distillation, and lactic acid and acrylic acid included in the azeotropic distillation column 200 were separated by the second lactic acid separation column 400 through distillation.

Meanwhile, acrylic acid was obtained through the upper discharge of the second lactic acid separation column 400, and in order to achieve the same acrylic acid content (98%) as in Example 1, the energy required in the first and second lactic acid separation columns 300 and 400 were 1.65 Gcal/hr and 0.18 Gcal/hr, respectively (1 Gcal/hr = 4.184 GJ/hr).

The flow rate (kg/hr) and composition (wt%) of each component in each stream in Comparative Example 1 are shown in Table 3.

**[Table 3]**

| Component | Unit | 1 | 2 | 3 | 4 | 5 | 6 | 7 | 8 |
|---|---|---|---|---|---|---|---|---|---|
| Flow rate | | 11000 | 1023 | 9977 | 4275 | 2669 | 811 | 1737 | 485 |
| N₂ | kg/hr | 1000 | 1000 | 0 | 0 | 0 | 0 | 0 | 0 |
| Lactic acid | kg/hr | 600 | 0 | 600 | 0 | 0 | 246 | 37 | 317 |
| Water | kg/hr | 7480 | 22 | 7458 | 4275 | 2622 | 555 | 6 | 0 |
| Acrylic acid | kg/hr | 1920 | 1 | 1919 | 0 | 46 | 10 | 1694 | 168 |
| Composition | | | | | | | | | |
| N₂ | wt% | 9% | 98% | 0% | 0% | 0% | 0% | 0% | 0% |
| Lactic acid | wt% | 5% | 0% | 6% | 0% | 0% | 30% | 2% | 65% |
| Water | wt% | 68% | 2% | 75% | 100% | 98% | 68% | 0% | 0% |
| Acrylic acid | wt% | 17% | 0% | 19% | 0% | 2% | 1% | 98% | 35% |

### Comparative Example 2

According to the process flow chart illustrated in FIG. 2, the acrylic acid producing process was simulated using Aspen Plus simulator available from Aspen Technology, Inc.

Specifically, a process was performed in the same manner as that of Comparative Example 1, except that 40 wt% of a lactic acid aqueous solution was supplied to a reactor, and the lower temperature of the cooling tower 10 was controlled to 109°C.

In the case of Comparative Example 2, in order to achieve the same acrylic acid content (88 wt%) in the stream from which acrylic acid was recovered as in Example 2 in which 40 wt% of the lactic acid aqueous solution was supplied, the energy required in the first and second lactic acid separation columns 300 and 400 were 1.35 Gcal/hr and 0.16 Gcal/hr, respectively (1 Gcal/hr = 4.184 GJ/hr).

The flow rate (kg/hr) and composition (wt%) of each component in each stream in Comparative Example 2 are shown in Table 4.

**[Table 4]**

| Component | Unit | 1 | 2 | 3 | 4 | 5 | 6 | 7 | 8 |
|---|---|---|---|---|---|---|---|---|---|
| Flow rate | | 11000 | 1023 | 9977 | 4482 | 983 | 1412 | 1567 | 1533 |
| N₂ | kg/hr | 1000 | 1000 | 0 | 0 | 0 | 0 | 0 | 0 |
| Lactic acid | kg/hr | 2000 | 0 | 2000 | 0 | 0 | 498 | 188 | 1314 |
| Water | kg/hr | 6400 | 22 | 6378 | 4481 | 983 | 914 | 0 | 0 |
| Acrylic acid | kg/hr | 1600 | 1 | 1599 | 1 | 0 | 0 | 1379 | 219 |
| Composition | | | | | | | | | |
| N₂ | wt% | 9% | 98% | 0% | 0% | 0% | 0% | 0% | 0% |
| Lactic acid | wt% | 18% | 0% | 20% | 0% | 0% | 35% | 12% | 86% |
| Water | wt% | 58% | 2% | 64% | 100% | 100% | 65% | 0% | 0% |
| Acrylic acid | wt% | 15% | 0% | 16% | 0% | 0% | 0% | 88% | 14% |

That is, in Example 1 and Comparative Example 1, the concentration of the lactic acid aqueous solution for the dehydration reaction of lactic acid was 30 wt%. Referring to Tables 1 and 3, in the case where unreacted lactic acid and a light gas component were separated using two cooling towers as in Example 1, the total process energy to achieve the same acrylic acid content (98 wt%) as in Comparative Example 1 was reduced. Specifically, in Comparative Example 1, energy of 1.83 Gcal/hr was required to operate an additional distillation tower for recovering unreacted lactic acid, which required for energy of about 3.3 Gcal/ton for unreacted lactic acid (flow rate: 0.563 ton/hr) to be recovered (1 Gcal/hr = 4.184 GJ/hr).

Similarly, in Example 2 and Comparative Example 2, the concentration of the lactic acid aqueous solution for the dehydration reaction of lactic acid was 40 wt%. Referring to Tables 2 and 4, in the case where unreacted lactic acid and a light gas component were separated using two cooling towers as in Example 2, the total process energy to achieve the same acrylic acid content (88 wt%) as in Comparative Example 2 was reduced. Specifically, in Comparative Example 2, energy of 1.51 Gcal/hr was required to operate an additional distillation tower for recovering unreacted lactic acid, which required an energy of about 0.8 Gcal/ton for unreacted lactic acid (flow rate: 1.812 ton/hr) to be recovered(1 Gcal/hr = 4.184 GJ/hr).

## Claims

1. A method for preparing acrylic acid, the method comprising:
obtaining a reaction product including unreacted lactic acid, water, a light gas component, and acrylic acid by supplying a lactic acid aqueous solution to a reactor to allow a dehydration reaction to proceed;
supplying the reaction product to a first cooling tower to separate the reaction product into a lower fraction containing unreacted lactic acid and an upper fraction containing water, light gas component, and acrylic acid;
supplying the upper fraction of the first cooling tower to a second cooling tower to separate a lower fraction containing water and acrylic acid therefrom; and
obtaining acrylic acid by purifying the lower fraction of the second cooling tower
wherein an operating temperature of the first cooling tower is 100°C to 180°C,
wherein an operating temperature of the second cooling tower is 60°C to 140°C,
wherein the dehydration reaction is performed as a gas phase reaction in the presence of a catalyst.

2. The method of claim 1, wherein an operating pressure of the first cooling tower is 98 kPa to 1960 kPa (1 kg/cm² to 20 kg/cm²).

3. The method of claim 1, wherein an amount of unreacted lactic acid contained in the upper fraction discharged from the first cooling tower is 5 wt% or less.

4. The method of claim 1, wherein operating pressure of the second cooling tower is 78.4 kPa to 1960 kPa (0.8 kg/cm² to 20 kg/cm²).

5. The method of claim 1, wherein unreacted lactic acid is recovered from the lower fraction of the first cooling tower, and the recovered unreacted lactic acid is circulated to the reactor.

6. The method of claim 1, wherein a ratio of a flow rate of acrylic acid contained in a lower discharge stream of the first cooling tower to a flow rate of acrylic acid included in the reaction product is 15 wt% or less.

7. The method of claim 1, wherein the light gas component is separated through the upper of the second cooling tower.

8. The method of claim 1, wherein a part of the lower fraction of the second cooling tower is supplied to an extraction column as a first acrylic acid aqueous solution stream, and a remainder is supplied to an azeotropic distillation column as a second acrylic acid aqueous solution stream,
an extract containing acrylic acid and an extraction solvent obtained from the extraction column is supplied to the azeotropic distillation column, and
acrylic acid is obtained from a lower fraction of the azeotropic distillation column.

9. The method of claim 8, wherein a ratio of a mass flow rate of the first acrylic acid aqueous solution stream supplied to the extraction column to the total flow rate of the first acrylic acid aqueous solution stream and the second acrylic acid aqueous solution stream is 30 wt% to 70 wt%.

10. The method of claim 8, wherein an upper fraction of the azeotropic distillation column is supplied to a layer separator to separate water and an extraction solvent, and the separated extraction solvent is circulated to one or more of the extraction column and the azeotropic distillation column.

## Patentansprüche

1. Verfahren zur Herstellung von Acrylsäure, wobei das Verfahren umfasst:
Erhalten eines Reaktionsprodukts, das nicht umgesetzte Milchsäure, Wasser, eine Leichtgaskomponente und Acrylsäure enthält, durch Zuführen einer wässrigen Milchsäurelösung zu einem Reaktor, um eine Dehydratisierungsreaktion ablaufen zu lassen;
Zuführen des Reaktionsprodukts zu einem ersten Kühlturm, um das Reaktionsprodukt in eine untere Fraktion, die nicht umgesetzte Milchsäure enthält, und eine obere Fraktion, die Wasser, eine Leichtgaskomponente und Acrylsäure enthält, zu trennen;
Zuführen der oberen Fraktion des ersten Kühlturms zu einem zweiten Kühlturm, um eine untere Fraktion, die Wasser und Acrylsäure enthält, davon zu trennen; und
Erhalten von Acrylsäure durch Reinigen der unteren Fraktion des zweiten Kühlturms,
wobei eine Betriebstemperatur des ersten Kühlturms 100 °C bis 180 °C beträgt,
wobei eine Betriebstemperatur des zweiten Kühlturms 60 °C bis 140 °C beträgt,
wobei die Dehydratisierungsreaktion als eine Gasphasenreaktion in Gegenwart eines Katalysators durchgeführt wird.

2. Verfahren nach Anspruch 1, wobei ein Betriebsdruck des ersten Kühlturms 98 kPa bis 1960 kPa (1 kg/cm² bis 20 kg/cm²) beträgt.

3. Verfahren nach Anspruch 1, wobei eine Menge an nicht umgesetzter Milchsäure, die in der aus dem ersten Kühlturm ausgetragenen oberen Fraktion enthalten ist, 5 Gew.-% oder weniger beträgt.

4. Verfahren nach Anspruch 1, wobei ein Betriebsdruck des zweiten Kühlturms 78,4 kPa bis 1960 kPa (0,8 kg/cm² bis 20 kg/cm²) beträgt.

5. Verfahren nach Anspruch 1, wobei nicht umgesetzte Milchsäure aus der unteren Fraktion des ersten Kühlturms gewonnen wird und die gewonnene nicht umgesetzte Milchsäure zu dem Reaktor zirkuliert wird.

6. Verfahren nach Anspruch 1, wobei ein Verhältnis einer Strömungsrate von Acrylsäure, die in einem unteren Austragsstrom des ersten Kühlturms enthalten ist, zu einer Strömungsrate von Acrylsäure, die in dem Reaktionsprodukt enthalten ist, 15 Gew.-% oder weniger beträgt.

7. Verfahren nach Anspruch 1, wobei die Leichtgaskomponente durch die Oberseite des zweiten Kühlturms getrennt wird.

8. Verfahren nach Anspruch 1, wobei ein Teil der unteren Fraktion des zweiten Kühlturms einer Extraktionskolonne als ein erster wässriger Acrylsäurelösungsstrom zugeführt wird und ein Rest einer azeotropen Destillationskolonne als ein zweiter wässriger Acrylsäurelösungsstrom zugeführt wird,
ein Extrakt, der Acrylsäure und ein Extraktionslösungsmittel enthält, das aus der Extraktionskolonne erhalten wird, der azeotropen Destillationskolonne zugeführt wird und
Acrylsäure aus einer unteren Fraktion der azeotropen Destillationskolonne erhalten wird.

9. Verfahren nach Anspruch 8, wobei ein Verhältnis einer Massenströmungsrate des ersten wässrigen Acrylsäurelösungsstroms, der der Extraktionskolonne zugeführt wird, zu der Gesamtströmungsrate des ersten wässrigen Acrylsäurelösungsstroms und des zweiten wässrigen Acrylsäurelösungsstroms 30 Gew.-% bis 70 Gew.-% beträgt.

10. Verfahren nach Anspruch 8, wobei eine obere Fraktion der azeotropen Destillationskolonne einem Schichttrenner zugeführt wird, um Wasser und ein Extraktionslösungsmittel zu trennen, und das getrennte Extraktionslösungsmittel zu einer oder mehreren der Extraktionskolonne und der azeotropen Destillationskolonne zirkuliert wird.

## Revendications

1. Procédé de préparation d'acide acrylique, le procédé comprenant :
obtenir un produit de réaction comprenant de l'acide lactique inaltéré, de l'eau, un composant de gaz léger et de l'acide acrylique en fournissant une solution aqueuse d'acide lactique à un réacteur pour permettre à une réaction de déshydratation de se faire ;
fournir le produit de réaction à une première tour de refroidissement pour séparer le produit de réaction en une fraction inférieure contenant l'acide lactique inaltéré et une fraction supérieure contenant l'eau, le composant de gaz léger et l'acide acrylique ;
fournir la fraction supérieure de la première tour de refroidissement à une deuxième tour de refroidissement pour séparer de celle-ci une fraction inférieure contenant l'eau et l'acide acrylique; et
obtenir l'acide acrylique en purifiant la fraction inférieure de la deuxième tour de refroidissement,
dans lequel une température de fonctionnement de la première tour de refroidissement est de 100 °C à 180 °C,
dans lequel une température de fonctionnement de la deuxième tour de refroidissement est de 60 °C à 140 °C.
dans lequel la réaction de déshydratation est effectuée comme une réaction en phase gazeuse en présence d'un catalyseur.

2. Procédé selon la revendication 1, dans lequel une pression de fonctionnement de la première tour de refroidissement est de 98 kPa à 1960 kPa (1 kg/cm² à 20 kg/cm²).

3. Procédé selon la revendication 1, dans lequel une quantité d'acide lactique inaltéré contenu dans la fraction supérieure déchargée de la première tour de refroidissement est de 5 % ou moins en poids.

4. Procédé selon la revendication 1, dans lequel une pression de fonctionnement de la deuxième tour de refroidissement est de 78,4 kPa à 1960 kPa (0,8 kg/cm² à 20 kg/cm²).

5. Procédé selon la revendication 1, dans lequel l'acide lactique inaltéré est récupéré de la fraction inférieure de la première tour de refroidissement, et l'acide lactique inaltéré récupéré est circulé vers le réacteur.

6. Procédé selon la revendication 1, dans lequel un rapport d'un débit d'acide acrylique contenu dans un courant de décharge inférieur de la première tour de refroidissement à un débit d'acide acrylique inclus dans le produit de réaction est de 15 % ou moins en poids.

7. Procédé selon la revendication 1, dans lequel le composant de gaz léger est séparé par la partie supérieure de la deuxième tour de refroidissement.

8. Procédé selon la revendication 1, dans lequel une partie de la fraction inférieure de la deuxième tour de refroidissement est fournie à une colonne d'extraction en tant que premier courant de solution aqueuse d'acide acrylique et le reste est fourni à une colonne de distillation azéotropique en tant que deuxième courant de solution aqueuse d'acide acrylique,
un extrait contenant l'acide acrylique et un solvant d'extraction obtenus de la colonne d'extraction est fourni à la colonne de distillation azéotropique, et
l'acide acrylique est obtenu d'une fraction inférieure de la colonne de distillation azéotropique.

9. Procédé selon la revendication 8, dans lequel un rapport d'un débit massique du premier courant de solution aqueuse d'acide acrylique fourni à la colonne d'extraction au débit total du premier courant de solution aqueuse d'acide acrylique et du deuxième courant de solution aqueuse d'acide acrylique est de 30 % en poids à 70 % en poids.

10. Procédé selon la revendication 8, dans lequel une fraction supérieure de la colonne de distillation azéotropique est fournie à un séparateur de couches pour séparer l'eau et un solvant d'extraction et le solvant d'extraction séparé est circulé vers une ou plusieurs d'entre la colonne d'extraction et la colonne de distillation azéotropique.
